(19)

**Europäisches Patentamt**
**European Patent Office**
**Office européen des brevets**

(11)  **EP 4 113 096 B1**

(12)  **EUROPEAN PATENT SPECIFICATION**

(45) Date of publication and mention
of the grant of the patent:
**22.04.2026  Bulletin 2026/17**

(21) Application number: **21803815.6**

(22) Date of filing: **18.03.2021**

(51) International Patent Classification (IPC):
*G01N 1/22* *(2006.01)*     *G01N 7/16* *(2006.01)*
*G01N 33/00* *(2006.01)*    *G01N 27/407* *(2006.01)*
*G01N 33/28* *(2006.01)*    *G01N 1/44* *(2006.01)*

(52) Cooperative Patent Classification (CPC):
**G01N 33/005; G01N 1/2226; G01N 27/4074;**
**G01N 33/0009; G01N 33/2841;** G01N 2001/2229

(86) International application number:
**PCT/KR2021/003369**

(87) International publication number:
**WO 2021/230484 (18.11.2021 Gazette 2021/46)**

(54) **GAS SENSING DEVICE INCLUDING HOUSING HAVING CONNECTION PASSAGE**

GASERFASSUNGSVORRICHTUNG MIT GEHÄUSE MIT VERBINDUNGSDURCHGANG

DISPOSITIF DE DÉTECTION DE GAZ COMPRENANT UN BOÎTIER AYANT UN PASSAGE DE
RACCORDEMENT

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority:  **12.05.2020  KR 20200056689**

(43) Date of publication of application:
**04.01.2023  Bulletin 2023/01**

(73) Proprietor: **PSS Inc.**
**Daejeon 34141 (KR)**

(72) Inventor: **PARK, Chong Ook**
**Daejeon 34141 (KR)**

(74) Representative: **Mammel und Maser**
**Patentanwälte**
**PartG mbB**
**Tilsiter Straße 3**
**71065 Sindelfingen (DE)**

(56) References cited:
JP-A- 2012 127 668      JP-A- 2017 058 358
KR-A- 20140 026 583     KR-A- 20140 054 545
KR-A- 20180 004 490     US-A1- 2004 182 705
US-A1- 2016 231 303     US-A1- 2018 217 020
US-A1- 2020 064 301

## Description

### Technical Field

The present disclosure relates to a gas sensing device. More particularly, the present disclosure relates to a gas sensing device having a housing in which a connection passage is formed.

### Background Art

There is a situation in which a method of measuring a concentration of a dissolved gas dissolved in a liquid is used so as to detect characteristics or a change in the characteristics of the liquid. For example, in oils used in various mechanical devices, such as an engine oil of a vehicle, an insulating oil of a transformer, and so on, as deterioration progresses, a change in a concentration of a dissolved gas, such as an increase in hydrogen, carbon monoxide, acetylene gas, and so on, occurs. Therefore, when the concentration of the dissolved gas is measured, whether the oil is deteriorated may be sensed. In actuality, regarding the insulating oil of the transformer, it is reported that there is a risk of explosion when 1000 ppm or more of dissolved hydrogen is generated. In addition, in the field of nuclear power generation, by measuring a concentration of oxygen or deuterium that is dissolved in water, information about corrosion of a pipe or information about power generation may be known. Further, in the metal industry, by measuring a concentration of a dissolved gas in a molten metal, quality of a manufactured metal may be constantly maintained and managed.

In order to measure a concentration of a dissolved gas, a method in which a dissolved gas is extracted from a liquid sample after the liquid sample is collected and then the dissolved gas is analyzed by a gas chromatography is generally used. However, there is a limitation that this method is not capable of measuring a concentration of a dissolved gas in real time in the industrial field.

In Korean Patent No. 10-1512189, a technology of measuring a concentration of a dissolved hydrogen gas by inserting a hydrogen sensor element, including a sensor unit that uses a solid electrolyte, into an oil has been proposed. In this technology, there is an advantage that a concentration of a dissolved gas may be easily measured in real time, but there is a problem that a sensing electrode of the sensor unit may be easily deteriorated since the sensing electrode is in direct contact with a liquid.

In Korean Patent Application Publication No. 2016-0011722, a technology of measuring a concentration of a dissolved hydrogen gas has been proposed. In this technology, a hydrogen sensor element in which a sensor unit is disposed in a sealed space formed by a housing and a gas separation membrane is inserted into a liquid, so that a sensing electrode of the sensor unit is not directly exposed to the liquid and the concentration of the dissolved hydrogen gas permeated in the sealed space through the gas separation membrane is measured. In this technology, there is an advantage that a concentration of a dissolved gas may be easily measured in real time while deterioration of the sensing electrode of the sensor unit is restrained.

However, when the sensing electrode of the sensor unit is disposed in the sealed space, evaporation of the dissolved gas in the sealed space and movement thereof to the sensing electrode of the sensor unit may be difficult due to a pressure in the sealed space. Particularly, a gas sensor is generally operated in a state in which the gas sensor is heated to a high temperature by using a heater, so that the pressure inside the sealed space in which the sensing electrode of the sensor unit is disposed is further increased. Therefore, evaporation of the dissolved gas and movement thereof from the liquid to a position of the sensing electrode of the sensor unit may be difficult, which may be an obstacle in rapidly and accurately measuring the concentration of the dissolved gas.

In order to solve this problem, a method of communicating external air to a space inside the housing where the sensing electrode of the sensor unit is disposed may be considered so that the pressure does not increase above atmospheric pressure even if the sensor unit is heated to a high temperature. However, in this situation, since the dissolved gas that is evaporated from the liquid is discharged to external air, there is a problem that it is difficult to be considered that the concentration of a gas in the space where the sensing electrode of the sensor unit is disposed accurately represents the concentration of the dissolved gas in the liquid.

US 2004/018705 A1 discloses a gas sensor comprising a housing with an opening part, through which a target gas to be sensed enters in an inner space of the housing. The space is in communication with external air only through the opening part. A sensor unit is disposed in the inner space of the housing. A connection passage having a first opening and a second opening is formed in the housing and the first opening and the second opening are open towards the inner space of the housing.

US 2018/0217020 A1 further discloses a sensor unit and an airtightness inspection device. There is an inlet opening through which external air passes into the inner space for passing through the gas sensor and subsequently flowing to one of both outlets, which is opened.

### Disclosure

**Technical Problem**

Accordingly, the present disclosure has been made keeping in mind the above problems occurring in the related art, and an objective of the present disclosure is to provide a gas sensing device capable of measuring a concentration of a gas with a high response speed and a high accuracy even if a pressure of a space inside a housing where a sensor unit is disposed increases.

The objective of the present disclosure is not limited thereto, and other objectives and other advantages of the present disclosure will be understood from the following description.

**Technical Solution**

The technical solution is provided by the appended set of claims.

In order to achieve the above objective, according to an embodiment of the present disclosure, there is provided a gas sensing device including: a housing including an opening part through which a target gas to be sensed enters an inner space thereof; a sensor unit disposed in the inner space of the housing; and a connection passage connecting a first opening and a second opening that are formed in the housing such that the first opening and the second opening are open toward the inner space of the housing. Further, the gas sensing device may further include a heater unit for heating the sensor unit to a sensing temperature.

The inner space of the housing is in communication with external air only through the opening part.

The inner space of the housing includes a first inner space between the sensor unit and the opening part and a second inner space that is a space except for the first inner space, the first opening opens toward the first inner space, and the second opening opens toward the second inner space.

The housing may be formed in a hollow tubular shape such that the opening part is formed in a first end portion of the housing in a longitudinal direction, the sensor unit may be disposed at the inner space of the housing while the sensor unit is in a state of being fixed to a first end portion of a frame in a longitudinal direction, the frame having a diameter smaller than an inner diameter of the housing, and a second end portion of the frame in the longitudinal direction may be gas-tightly fixed to the housing. Therefore, a space between the frame and an inner wall of the housing may form the second inner space, and a circulation path circulating the first inner space, the first opening, the connection passage, the second opening, and the second inner space may be formed.

In addition, the housing may be formed in a hollow tubular shape such that the opening part is formed in a first end portion of the housing in a longitudinal direction, a second end portion of the housing in the longitudinal direction may be blocked by a cover part, and the sensor unit may be disposed at the inner space of the housing while the sensor unit is in a state of being coupled to an inner wall of the housing. Here, a gap may exist between the sensor unit and the inner wall of the housing, so that a circulation path circulating the first inner space, the first opening, the connection passage, the second opening, and the second inner space may be formed.

The sensor unit may include a hydrogen sensor element, and the hydrogen sensor element may include: a solid electrolyte; a sensing electrode formed on a first surface of the solid electrolyte in a direction toward the opening part; and a reference electrode formed on a second surface of the solid electrolyte, wherein the second surface is an opposite surface of the electrolyte to the first surface, wherein the first opening may be positioned between the sensing electrode and the opening part.

According to an embodiment of the present disclosure, there is provided a gas sensing device including: a housing which includes an inner space and which is formed in a hollow tubular shape, the housing being configured such that a lower end portion of the housing in a longitudinal direction is open toward the inner space, thereby forming an opening part such that a target gas to be sensed enters the inner space; a sensor unit disposed at a position of the inner space, the position being spaced apart from both an upper end portion and the lower end portion of the housing in the longitudinal direction by a predetermined distance, the sensor unit including a sensing electrode formed to face the opening part; and a heater unit provided to heat the sensor unit to a sensing temperature, wherein the inner space of the housing may include a first inner space and a second inner space that are respectively positioned below and above the sensing electrode of the sensor unit on the basis of the sensing electrode of the sensor unit, a first opening formed to be open toward the first inner space and a second opening formed to be open toward the second inner space may be formed in the housing, and a connection passage connecting the first opening and the second opening may be provided, thereby forming a circulation path in which the target gas to be sensed enters through the opening part and which is circulated through the first inner space, the first opening, the connection passage, the second opening, and the second inner space.

Here, the sensor unit may be disposed at the inner space while the sensor unit is in a state of being fixed to a lower end portion of a frame which is formed in a hollow tubular shape and which has a diameter smaller than an inner diameter of the housing, an inner portion of the frame may be exposed to external air while the inner portion of the frame is isolated from the inner space of the housing, and the sensor unit may further include a reference electrode that is exposed to the external air through the inner portion of the frame.

Otherwise, the sensor unit may be disposed at the inner space in a manner that a partial area of a border of the sensor unit is coupled to an inner wall of the housing through a predetermined coupling part, and a gap may be formed at a portion which is a position where the coupling part is not formed and which is positioned between the sensor unit and the inner wall of the housing, so that the first inner space and the second inner space may be in communication with each other through the gap.

## Advantageous Effects

According to the present disclosure, since the connection passage that connects openings formed in the housing to each other so that the openings are respectively open toward an upper inner space and a lower inner space of the sensor unit, there is an effect that the gas sensing device capable of measuring a concentration of a gas with high response speed and high accuracy may be provided even if a pressure of the inner space of the housing where the sensor unit is disposed increases.

However, the effect of the present disclosure is not limited to those mentioned above, and other effects not mentioned will be clearly understood by those skilled in the art from the following description.

## Description of Drawings

FIG. 1 is a cross-sectional view schematically illustrating a gas sensing device according to a first embodiment of the present disclosure.

FIG. 2 is a cross-sectional view schematically illustrating the gas sensing device according to a second embodiment of the present disclosure.

FIG. 3 is a cross-sectional view taken along line A-A in FIG. 2.

FIGS. 4 to 6 are views illustrating a hydrogen sensor element capable of being used as a sensor unit in the present disclosure.

FIGS. 7 and 8 are views illustrating examples of use of the gas sensing device according to an embodiment of the present disclosure.

FIG. 9 is a graph illustrating a measuring result of a concentration of a gas by using the gas sensing device according to an embodiment of the present disclosure.

FIG. 10 is a graph illustrating a measuring result of a concentration of a gas by using the gas sensing device according to a comparative example of the present disclosure.

## Best Mode

Hereinafter, embodiments of the present disclosure will be described in detail with reference to the accompanying drawings, but the present disclosure is not limited or restricted to the embodiments. In describing the embodiments of the present disclosure, corresponding components will be described with the same name and the same reference numeral. In describing the present disclosure, a detailed description of known technologies will be omitted when it may obscure the subject matter of the present disclosure. Unless otherwise defined, terms used in this specification should be interpreted as content commonly understood by those of ordinary skill in the art.

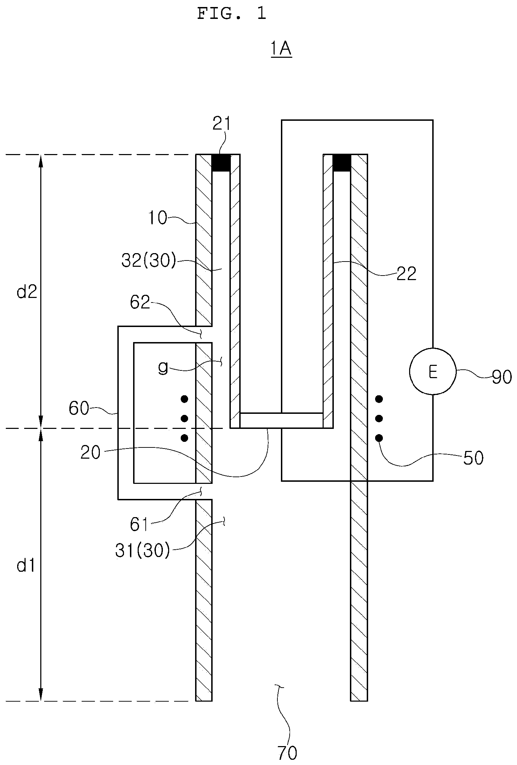

FIG. 1 is a cross-sectional view schematically illustrating a gas sensing device according to a first embodiment of the present disclosure. Referring to FIG. 1, a gas sensing device 1A according to a first embodiment of the present disclosure includes a housing 10 having a first side provided with an opening part 70, a sensor unit 20 disposed in the housing 10, a heater unit 50 for heating the sensor unit 20 to a sensing temperature, and a connection passage 60 connecting a first opening 61 and a second opening 62 that are formed in the housing 10.

The housing 10 may be provided in a hollow tubular shape. The opening part 70 may be formed in the first side (a lower side of FIG. 1) of the housing 10, and may provide an entrance into which a target gas to be sensed is injected. When the gas sensing device 1A is used for measuring a concentration of a gas in a measurement environment, the housing 10 may be mounted such that the opening part 70 is in communication with the measurement environment. For example, when the gas sensing device 1A is used for measuring a concentration of a dissolved gas in a liquid, the housing 10 of the gas sensing device 1A may be mounted such that the opening part 70 is in communication with a liquid storage container, or may be mounted such that a first end of the housing 10 where the opening part 70 is formed is submerged in the liquid. A dissolved gas evaporated from the liquid may be filled in an inner space 30 of the housing 10, and the sensor unit 20 may sense the dissolved gas.

The sensor unit 20 is disposed in the housing 10, and may be disposed at a position spaced apart from opposite end portions of the housing 10 in a longitudinal direction (a vertical direction of FIG. 1) of the housing 10. For example, as illustrated in FIG. 1, the sensor unit 20 may be disposed at a position which is spaced apart from a first end portion of the housing 10 at the opening part 70 side by a d1 distance in the longitudinal direction and which is spaced apart from a second end portion of the housing 10 by a d2 distance in the longitudinal direction. Here, the position of the sensor unit 20 may be a position of a sensing electrode that is formed at the sensor unit 20.

In order for disposing the sensor unit 20 at a position spaced apart from the end portions of the housing 10 in the longitudinal direction, the sensor unit 20 may be mounted in the housing 10 while being in a state in which the sensor unit 20 is fixed to a first end (the lower side of FIG. 1) of a frame 22 that has a predetermined length. The frame 22 is formed in a tubular shape having a diameter smaller than a diameter of the housing 10, so that a gap g may be formed between the frame 22 and an inner wall of the housing 10 in a width direction (a horizontal direction of FIG. 1). The sensor unit 20 may be fixed to a first end portion of the frame 22, and a second end portion of the frame 22 may be coupled to the housing 10. When the sensor unit 20 is formed in a tubular shape, the frame 22 may be a component included in the sensor unit 20. Various coupling methods such as adhesive coupling, screw coupling, brazing, and so on may be used to couple the frame 22 to the housing 10, and a coupling method is not limited to a specific coupling method. Meanwhile, the frame 22 may be coupled to the housing 10 by a gas-tight coupling method. To this end, a sealing material 21 may be included at a coupling portion where the frame 22 and the housing 10 are coupled to each other. The sealing material 21 may be formed of an elastic polymer material such as an O-ring. Otherwise, when the frame 22 is coupled to the housing 10 by the adhesive coupling method, the sealing material 21 may be an adhesive material.

Since the housing 10 and the frame 22 are coupled to each other such that the housing 10 and the frame 22 are gas-tightly coupled to each other, the inner space 30 of the housing 10 except for the opening part 70 may form a sealed space blocked from external air. Otherwise, when the second side (an upper portion of FIG. 1) of the housing 10 where the opening part 70 is not formed is formed in a confined structure, the sealed space is capable of being formed in the inner space 30 even if the frame 22 is not gas-tightly coupled to the housing 10.

The inner space 30 of the housing 10 is divided into a first inner space 31 and a second inner space 32 by a position where the sensor unit 20 is mounted. In the inner space 30 of the housing 10, the first inner space 31 is a space between the sensor unit 20 and the opening part 70. Further, in the inner space 30 of the housing, the second inner space 32 is a space except for the first inner space 31. In an embodiment in FIG. 1, the first inner space 31 may be an inner space below the sensor unit 20, and the second inner space 32 may be an inner space above the sensor unit 20. That is, the second inner space 32 may be a space formed by the gap g between the frame 22 and the inner wall of the housing 10. Here, an upper side and a lower side of the sensor unit 20 may be divided on the basis of the position of the sensing electrode that is formed at the sensor unit 20.

The heater unit 50 is a configuration for heating the sensor unit 20 to the sensing temperature. The sensing temperature may vary according to a type of a sensor, and may be at least 300°C. As exemplarily illustrated in FIG. 1, the heater unit 50 may include a resistive heating type heating coil that is wound on an outer portion of the housing 10 where the sensor unit 20 is disposed, but may be provided in various types that are not limited thereto. For example, the heater unit 50 may be disposed inside the housing 10, and may be provided in a heating pattern shape printed on a predetermined substrate, instead of the heating coil. Otherwise, the heater unit 50 may be provided as the heating coil wound on the frame 22, or may be provided such that the heater unit 50 is mounted inside the frame 22 or the sensor unit 20. The heater unit 50 is not limited to the resistive heating type, and may be provided as a light irradiation type heater unit such as a heating lamp, an LED, and so on.

When the sensor unit 20 does not required to be heated since the gas sensing device 1A is used in a high temperature environment, the heater unit 50 may be omitted. For example, when the gas sensing device 1A is used for measuring a concentration of a dissolved gas in a high temperature molten metal, the gas sensing device 1A may not include the heater unit 50.

When the gas sensing device 1A is connected with a high pressure measurement environment or when the opening part 70 is connected to a liquid storage container so as to measure a dissolved gas in a liquid, the inner space 30 may become a high pressure space. Particularly, when the sensor unit 20 is heated to a high temperature by using the heater unit 50, a pressure of the inner space 30 may further increase according to a temperature. Due to such an increase in pressure, a target gas to be sensed may be difficult to be moved to the sensor unit 20.

In order for solving this problem, the gas sensing device 1A according to the first embodiment of the present disclosure forms the connection passage 60. Specifically, the first opening 61 and the second opening 62 are formed in the housing 10, and the first opening 61 and the second opening 62 are connected to each other by the connection passage 60. The first opening 61 and the second opening 62 is formed in the housing 10 such that the first opening 61 is open toward the first inner space 31 and the second opening 62 is open toward the second inner space 32. Referring to FIG. 1, the first opening 61 may be formed below the sensor unit 20, and the second opening 62 may be formed above the sensor unit 20. Here, the upper side and the lower side of the sensor unit 20 may be divided on the basis of the position of the sensing electrode that is formed at the sensor unit 20.

By this structure, a circulation path into which a gas at the first inner space 31 enters the first opening 61, the connection passage 60, the second opening 62, the second inner space 32, and the first inner space 31 may be formed again. By forming such a circulation path, even if a vicinity of the sensor unit 20 is in a high pressure state, the target gas which is to be sensed and which is injected into the inner space 30 through the opening part 70 may be more easily moved toward the sensor unit 20.

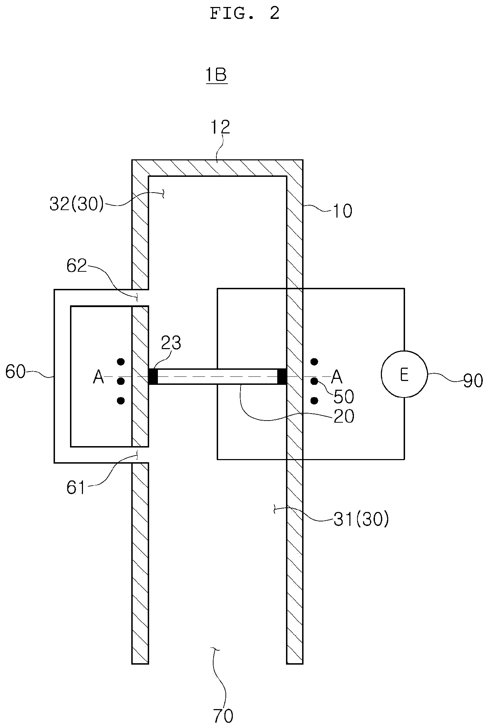

FIG. 2 is a cross-sectional view schematically illustrating the gas sensing device according to a second embodiment of the present disclosure. Referring to FIG. 2, a gas sensing device 1B according to the second embodiment of the present disclosure is different from the gas sensing device 1A according to the first embodiment in that the sensor unit 20 is disposed in the housing 10 without using a frame.

The gas sensing device 1B according to the second embodiment of the present disclosure includes the housing 10 having the first side provided with the opening part 70, the sensor unit 20 disposed in the housing 10, the heater unit 50 for heating the sensor unit 20 to the sensing temperature, and the connection passage 60 connecting the first opening 61 and the second opening 62 that are formed in the housing 10.

The housing 10 may be provided in a hollow tubular shape having an open first side (a lower side of FIG. 2). The open first side forms the opening part 70, and the second side (an upper side of FIG. 2) may have a structure confined by a cover part 12. Therefore, except for the opening part 70, a sealed space blocked from external air is formed in the inner space 30 of the housing 10.

The sensor unit 20 is disposed at the inner space 30 of the housing 10, and may be disposed at a position spaced apart from the opposite end portions of the housing 10 in the longitudinal direction (a vertical direction of FIG. 2). The sensor unit 20 may be coupled to the inner space 30 of the housing 10 by a coupling part 23. The coupling part 23 may be an adhesive material, but is not limited thereto. Further, various coupling means capable of disposing the sensor unit 20 at the inner space 30 of the housing 10 may be used.

The inner space 30 of the housing 10 is divided into the first inner space 31 and the second inner space 32 by a position where the sensor unit 20 is mounted. In the inner space 30 of the housing 10, the first inner space 31 is a space between the sensor unit 20 and the opening part 70. Further, in the inner space 30 of the housing, the second inner space 32 is a space except for the first inner space 31. In an embodiment in FIG. 2, the first inner space 31 may be an inner space below the sensor unit 20, and the second inner space 32 may be an inner space above the sensor unit 20. That is, the second inner space 32 may be a space between the cover part 12 and the sensor unit 20. Here, the upper side and the lower side of the sensor unit 20 may be divided on the basis of the position of the sensing electrode that is formed at the sensor unit 20.

As exemplarily illustrated in FIG. 2, the heater unit 50 is a component for heating the sensor unit 20 to the sensing temperature, and the heater unit 50 may include a resistive heating type heating coil that is wound on an outer portion of the housing 10 where the sensor unit 20 is disposed, but may be provided in various types that are not limited thereto. For example, the heater unit 50 may be disposed inside the housing 10, and may be provided in a heating pattern shape printed on a predetermined substrate, instead of the heating coil. Otherwise, the heater unit 50 may be provided such that the heater unit 50 is mounted inside the sensor unit 20. The heater unit 50 is not limited to the resistive heating type, and may be provided as a light irradiation type heater unit such as a heating lamp, an LED, and so on.

When the sensor unit 20 does not required to be heated since the gas sensing device 1B is used in a high temperature environment, the heater unit 50 may be omitted. For example, when the gas sensing device 1B is used for measuring a concentration of a dissolved gas in a high temperature molten metal, the gas sensing device 1B may not include the heater unit 50.

The first opening 61 and the second opening 62 are formed in the housing 10, and the first opening 61 and the second opening 62 are connected to each other by the connection passage 60. The first opening 61 and the second opening 62 is formed in the housing 10 such that the first opening 61 is open toward the first inner space 31 and the second opening 62 is open toward the second inner space 32. Referring to FIG. 2, the first opening 61 may be formed below the sensor unit 20, and the second opening 62 may be formed above the sensor unit 20. Here, the upper side and the lower side of the sensor unit 20 may be divided on the basis of the position of the sensing electrode that is formed at the sensor unit 20.

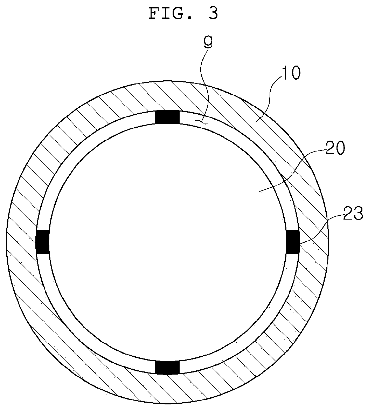

The sensor unit 20 is coupled to the housing 10 by the coupling part 23, but the first inner space 31 and the second inner space 32 may be not completely blocked by the coupling part 23 and the sensor unit 20. For example, the coupling part 23 may be provided such that the coupling part 23 couples only a partial area among a border of the sensor unit 20 to the inner wall of the housing 10, so that the gap may be formed between the sensor unit 20 and the inner wall of the housing 10. FIG. 3 is a view illustrating the configuration described above, and is a cross-sectional view taken along line A-A in FIG. 2. Referring to FIG. 3, the coupling part 23 is formed only on four positions in the border of the sensor unit 20. Further, in a position where the coupling part 23 is not formed, the predetermined gap g may be formed between the sensor unit 20 and the inner wall of the housing 10. Accordingly, a gas may be freely moved between the first inner space 31 and the second inner space 32. In addition to the coupling structure as described in FIG. 3, the first inner space 31 and the second inner space 32 may be in communication with each other by forming the coupling part 23 with an air-permeable material or by forming a through-hole (not illustrated) in the sensor unit 20.

By this structure, a circulation path into which a gas at the first inner space 31 enters the first opening 61, the connection passage 60, the second opening 62, the second inner space 32, and the first inner space 31 again may be formed. By forming such a circulation path, even if a vicinity of the sensor unit 20 is in a high pressure state, the target gas which is to be sensed and which is injected into the inner space 30 through the opening part 70 may be more easily moved toward the sensor unit 20.

In the sensor unit 20 used in the gas sensing device 1A or 1B according to embodiments of the present disclosure, various gas sensor elements may be used according to a target gas to be sensed, an application use, and so on. According to a target gas to be sensed, a hydrogen sensor element, a carbon monoxide sensor element, a hydrocarbon sensor element, and so on may be used as the sensor unit 20. In addition, according to the type of the sensor unit 20, various shapes of sensor elements, such as a pallet shape, a chip shape, a tube shape, and so on, may be used as the sensor unit 20. In addition, according to a gas detection principle, an electrochemical type sensor element that measures a change in electromotive force (EMF) according to a gas concentration by using a solid electrolyte, a semiconductor type sensor element using a semiconductor material in which an electrical resistance is changed according to a gas concentration, and so on may be used as the sensor unit 20.

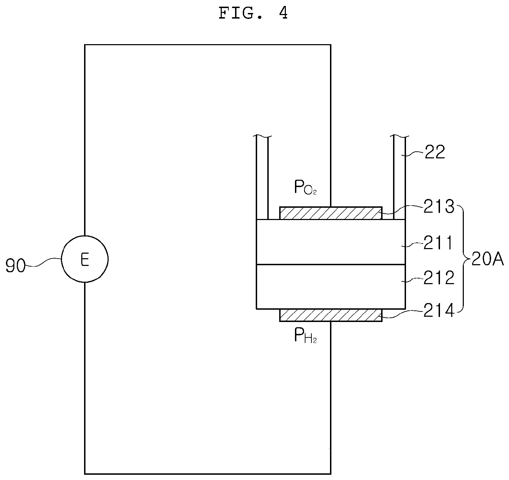

FIGS. 4 to 6 are views illustrating a hydrogen sensor element capable of being used as the sensor unit 20 in the present disclosure.

A sensor unit 20A in FIG. 4 is a hydrogen sensor element specifically suitable for use in a hydrogen sensing device according to the first embodiment (FIG. 1) of the present disclosure. Referring to FIG. 4, the sensor unit 20A may include a solid electrolyte having a heterojunction structure in which an oxygen ion conductor 211 and a hydrogen ion conductor 212 are joined, a reference electrode 213 formed on a surface of the oxygen ion conductor 211, and a sensing electrode 214 formed on a surface of the hydrogen ion conductor 212.

As the oxygen ion conductor 211, $CeO_2$ based compounds prepared by adding solid electrolyte or $Gd_2O_3$ may be used, such as stabilized zirconia prepared by adding various substances to zirconia $(ZrO_2)$, for example, Yttria stabilized zirconia (YSZ), calcium stabilized zirconia (CSZ), and magnesium stabilized zirconia (MSZ). As the hydrogen ion conductor 212, substances acquired by substituting a B position of a substance having an $ABO_3$ type perovskite structure with various substances, for example, $CaZrO_3$ based compounds including $CaZr_{0.9}In_{0.1}O_{3-x}$, and the like, $SrZrO_3$ based compounds including $SrZr_{0.95}Y_{0.05}O_{3-x}$, and the like, $BaCeO_3$ based compounds including $BaCe_{0.9}Nd_{0.1}O_{3-x}$, and the like, and Ti based compounds including $BaTiO_3$, $SrTiO_3$, $PbTiO_3$, and the like may be used.

In addition, the reference electrode 213 and the sensing electrode 214 may be formed of precious metal such as platinum (Pt), or the like.

The reference electrode 213 and the sensing electrode 214 are electrically connected to a measuring unit 90 through a lead wire, so that a concentration of a hydrogen gas may be measured by measuring an electromotive force. The electromotive force E measured between the reference electrode 213 and the sensing electrode 214 establishes the following relationship with oxygen partial pressure $P_{O2}$ at the reference electrode 213 side and hydrogen partial pressure $P_{H2}$ at the sensing electrode 214 side.

$$E = Eo + A \cdot \log P_{H2} + (A/2) \cdot \log P_{O2} \qquad -------- \quad (1)$$

Since Eo and A are constants that depend on only a temperature in the equation, consequently, when the oxygen partial pressure $P_{O2}$ at the reference electrode 213 side is known, it can be seen that the hydrogen partial pressure $P_{H2}$ at the sensing electrode 214 side may be decided by measuring the electromotive force E.

The oxygen partial pressure $P_{O2}$ at the reference electrode 213 side may be fixed by exposing the reference electrode 213 to the atmosphere. That is, referring to FIG. 1 and FIG. 4 together, by configuring the gas sensing device 1A such that the frame 22 fixed to the sensor unit 20A is gas-tightly coupled to the housing 10 by using the sealing material 21 and the reference electrode 213 is exposed to the atmosphere, the oxygen partial pressure $P_{O2}$ at the reference electrode 213 side may be fixed to 0.21 atmospheric pressure which is the oxygen partial pressure in the air. Therefore, when the electromotive force E between the reference electrode 213 and the sensing electrode 214 is measured, the hydrogen partial pressure $P_{H2}$ at the sensing electrode 214 side may be calculated by the Equation (1).

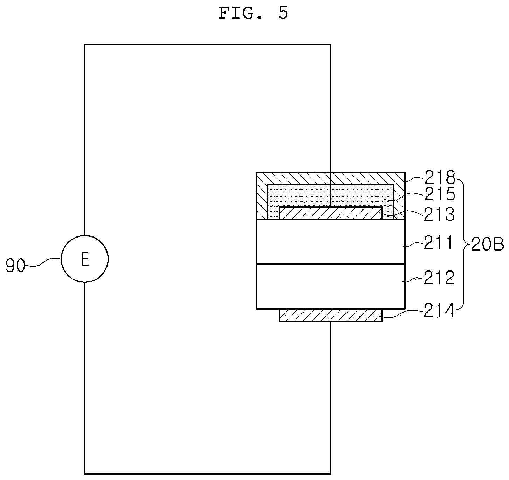

Instead of fixing the oxygen partial pressure $P_{O2}$ by exposing the reference electrode 213 to the atmosphere, a sensor unit 20B in FIG. 5 has a structure in which the oxygen partial pressure $P_{O2}$ is thermodynamically fixed by covering the reference electrode 213 with an oxygen partial pressure fixing reference substance 215. Therefore, the sensor unit 20B in FIG. 5 is different from the sensor unit 20A in FIG. 4.

As the oxygen partial pressure fixing reference substance 215, mixtures of metal and metal oxides, such as Cu/CuO, Ni/NiO, Ti/TiO$_2$, Fe/FeO, Cr/Cr$_2$O$_3$, Mo/MoO, and the like or mixtures of metal oxides having different oxidation degrees, such as Cu$_2$O/CuO, FeO/Fe$_2$O$_3$, and the like may be used. Further, when the reference electrode 213 is covered with the oxygen partial pressure fixing reference substance 215, the oxygen partial pressure at the reference electrode 213 side may be thermodynamically fixed. That is, the oxygen partial pressure at the reference electrode 213 side is decided by the oxygen partial pressure fixing reference substance 215 instead of the external air and similarly to the description referring to FIG. 4, the hydrogen partial pressure at the sensing electrode 214 side may be decided by the Equation (1) by measuring the electromotive force between the reference electrode 213 and the sensing electrode 214.

The sensor unit 20B in FIG. 5 may be suitable for either the gas sensing device 1A according to the first embodiment of the present disclosure or the gas sensing device 1B according to the second embodiment of the present disclosure. In order to prevent the oxygen partial pressure fixing reference substance 215 from being affected by external air or a gas atmosphere in the inner space 30, a sealing cover 218 for blocking the oxygen partial pressure fixing reference substance 215 from external air may be additionally provided. The sealing cover 218 may be formed of a dense ceramic material capable of preventing penetration of external air, and the like. Further, when the influence of external air is insignificant, the sealing cover 218 may be omitted.

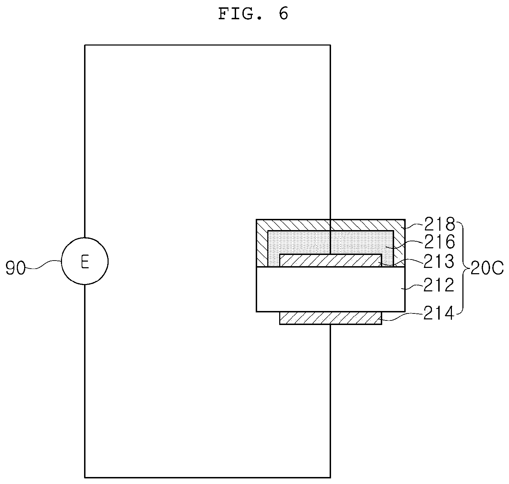

A sensor unit 20C in FIG. 6 includes the hydrogen ion conductor 212, the reference electrode 213 and the sensing electrode 214 that are respectively formed on opposite surfaces of the hydrogen ion conductor 212, and a hydrogen partial pressure fixing reference substance 216 covering the reference electrode 213. That is, comparing to the sensor unit 20B in FIG. 5, the sensor unit 20C in FIG. 6 has a difference in that the sensor unit 20C does not include the oxygen ion conductor and the reference electrode 213 is covered with the hydrogen partial pressure fixing reference substance 216 instead of the oxygen partial pressure fixing reference substance.

As the hydrogen partial pressure fixing reference substance 216, mixed phases of metal and metal hydrides, such as $Ti/TiH_2$, $Zr/ZrH_2$, $Ca/CaH_2$, $Nd/NdH_2$, and the like may be used. Therefore, a hydrogen partial pressure $P_{2, H2}$ at the reference electrode 213 side may be thermodynamically fixed by the mixed phases.

In the sensor unit 20C in FIG. 6, when the electromotive force E between the reference electrode 213 and the sensing electrode 214 is measured, a hydrogen partial pressure $P_{1, H2}$ at the sensing electrode 214 side may be decided by a Nernst equation given below.

$$E = -(RT/2F)\ln(P_{2, H2}/P_{1, H2}) \quad --------- \quad (2)$$

In Equation (2) given above, R represents a gas constant, F represents a Faraday constant, and T represents the sensing temperature and all of R, F, and T are constants. Further, since the hydrogen partial pressure $P_{2, H2}$ at the reference electrode 213 side is also a value decided by the hydrogen partial pressure fixing reference substance 216, the hydrogen partial pressure $P_{1, H2}$ at the sensing electrode 214 side may be calculated from the measured electromotive force E value.

The sensor unit 20C in FIG. 6 may be suitable for either the gas sensing device 1A according to the first embodiment of the present disclosure or the gas sensing device 1B according to the second embodiment of the present disclosure. In order to prevent the hydrogen partial pressure fixing reference substance 216 from being affected by external air or a gas atmosphere in the inner space 30, the sealing cover 218 for blocking the hydrogen partial pressure fixing reference substance 216 from external air may be additionally provided.

The gas sensing device according to embodiments of the present disclosure may be used for measuring a concentration of a dissolved gas in a liquid.

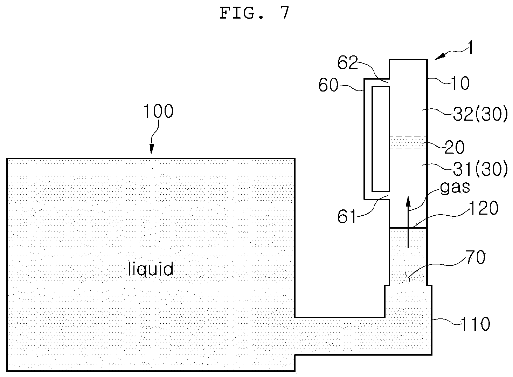

FIG. 7 is a view illustrating an example of use in which the gas sensing device of the present disclosure is used for measuring a concentration of a dissolved gas in a liquid stored in a liquid storage container 100. In FIG. 7, although a structure of a gas sensing device 1 is briefly illustrated mainly in main components, all of the gas sensing device 1A according to the first embodiment of the present disclosure or the gas sensing device 1B according to the second embodiment of the present disclosure may be used.

Referring to FIG. 7, the gas sensing device 1 according to the present disclosure may be coupled to the liquid storage container 100 via a connector 110. As illustrated in the drawing, the connector 110 may be formed on a side surface of the liquid storage container 100, but is not limited thereto. Further, the connector 110 may be formed on various positions such as an upper surface of the liquid storage container 100, a lower surface of the liquid storage container 100, and so on. When the gas sensing device 1 is coupled to the liquid storage container 100 via the connector 110, an inner portion of the liquid storage container 100 and the inner space 30 of the gas sensing device 1 are in communication with each other, and a dissolved gas in a liquid stored in the liquid storage container 100 may evaporate and be moved to the inner space 30.

Generally, a solubility of a gas in a liquid is in accordance with Sievert's law in Equation (3).

$$C = k \cdot P_{gas} \quad ---- \quad (3)$$

C is a concentration of a dissolved gas in a liquid, k is a constant that depends on the type of gas, a temperature, and so on, and $P_{gas}$ is a gas partial pressure in a space in contact with a liquid. That is, the concentration C of the dissolved gas and the gas partial pressure $P_{gas}$ in the space in contact with the liquid are in proportion to each other, and reach an equilibrium state when an evaporation speed of the gas that is evaporated from the liquid to the space is equal to a speed at which the gas inside the space is dissolved in the liquid.

According to this principle, when the gas sensing device 1 of the present disclosure is connected to the liquid storage container 100 as illustrated in FIG. 7, the dissolved gas in the liquid that is stored in the liquid storage container 100 is evaporated in the inner space 30 of the gas sensing device 1, and the equilibrium state is realized. Therefore, by measuring the concentration (gas partial pressure) of the gas in the inner space 30 by using the sensor unit 20, the concentration of the dissolved gas in the liquid may be known.

Meanwhile, according to a pressure of the liquid in the liquid storage container 100, a pressure inside the inner space 30 of the gas sensing device 1 that is connected to the liquid storage container 100 may increase. That is, the liquid stored in the liquid storage container 100 is moved to the inner space 30 of the gas sensing device 1 via the connector 110, so that a liquid surface 120 may be formed in the inner space 30 of the gas sensing device 1. Accordingly, a volume of the gas in the inner space 30 of the gas sensing device 1 may shrink until the pressure inside the inner space 30 is equal to the pressure of the liquid.

Due to such an increase in pressure inside the inner space 30, the gas evaporated from the liquid may be difficult to be moved to the sensing electrode 214 of the sensor unit 20. Particularly, when the sensor unit 20 is heated to the sensing temperature by using the heater unit 50, the pressure inside the inner space 30 is further increased, so that the dissolved gas may be further difficult to be moved to the sensing electrode 214. This situation may be an obstacle factor when the concentration of the dissolved gas is rapidly and accurately measured.

However, in the gas sensing device 1 according to the present disclosure, since the gas in the inner space 30 may be circulated along the connection passage 60 that connects the first opening 61 and the second opening 62 that are respectively open toward the first inner space 31 and the second inner space 32, the gas injected through the opening part 70 may be smoothly moved to the sensing electrode 214 even if the inner space 30 is in the high pressure state. Accordingly, the concentration of the dissolved gas is capable of being rapidly and accurately measured.

In FIG. 7, preferably, the liquid surface 120 is formed below the first opening 61, but the liquid surface 120 may be formed above the first opening 61. That is, even if the first opening 61 is submerged in the liquid, the dissolved gas evaporated from the liquid may be moved toward the second opening 62 through the connection passage 60. However, since the liquid surface 120 is required to be formed below the sensor unit 20, it is preferable that a length of the housing 10 and/or a height of the connector 110 is designed in consideration of a use environment such as a liquid pressure and so on. Optionally, a gas permeating filter may be disposed at the connector 110 or the first inner space 31 so as to prevent the sensor unit 20 from being contaminated by the liquid. If the gas permeating filter has a configuration in which a liquid does not pass through the gas permeating filter and only a gas can pass through the gas permeating filter, a material or a shape of the gas permeating filter is not particularly limited. For example, the gas permeating filter may be a gas permeating layer manufactured by including graphite, ceramic powder, a PTFE membrane, or the like.

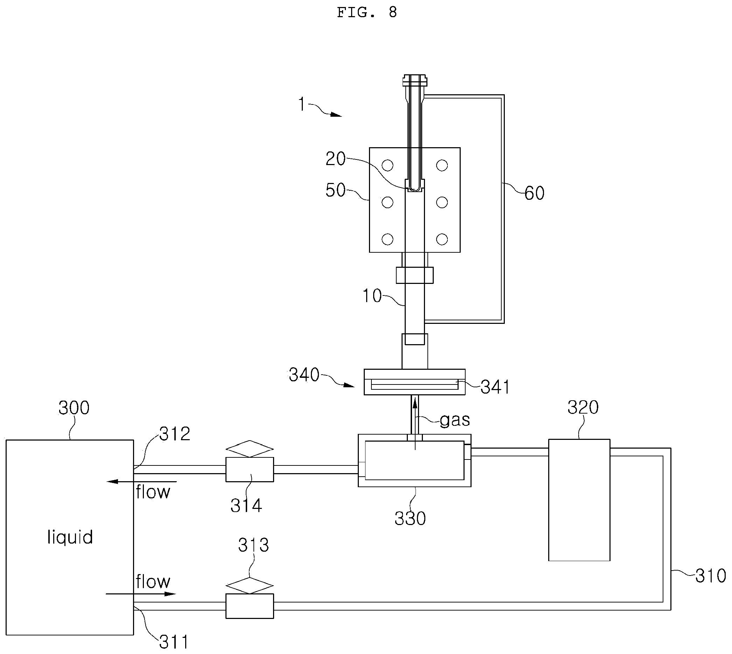

FIG. 8 is a view illustrating another example of use in which the gas sensing device of the present disclosure is used for measuring a concentration of a dissolved gas in a liquid stored in a liquid storage container 300. The liquid storage container 300 may be a transformer, and a liquid in the liquid storage container 300 may be an insulating oil of the transformer.

Referring to FIG. 8, a circulation pipe 310 for circulating a liquid is connected to the liquid storage container 300. The circulation pipe 310 is provided with valves 313 and 314 and a circulation motor 320. Further, in a state in which the valves 313 and 314 are open, when the circulation motor 320 is driven, a circulation path through which the liquid is circulated in a direction indicated by arrows in the drawing is formed.

A measurement tank 330 is provided on the circulation path, and the liquid circulated along the circulation pipe 310 is passing through the measurement tank 330. That is, the circulating liquid may be temporarily stored in the measurement tank 330.

The gas sensing device 1 according to the present disclosure may be coupled to the measurement tank 330 via the connector 340. Accordingly, the dissolved gas evaporated from the liquid in the measurement tank 330 may be moved to the inner space 30 of the gas sensing device 1. In order for preventing the liquid from moving to the gas sensing device 1, a gas permeating filter 341 may be provided in the connector 340. Either the gas sensing device 1A according to the first embodiment of the present disclosure or the gas sensing device 1B according to the second embodiment of the present disclosure may be used as the gas sensing device 1.

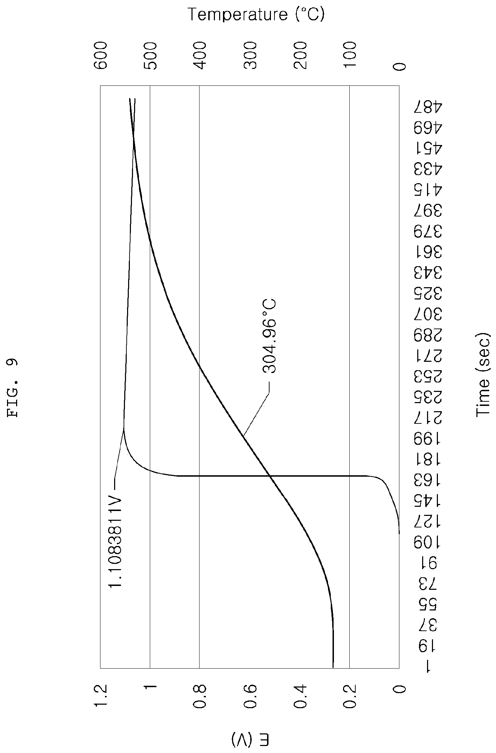

FIG. 9 is a graph illustrating a measuring result of the electromotive force E of the sensor unit 20 after the gas sensing device 1A according to the first embodiment of the present disclosure is connected with a measurement environment in which a hydrogen concentration is constantly maintained at 4%. At this time, the hydrogen sensor element in FIG. 4 was used as the sensor unit 20. That is, an electro-chemical hydrogen sensor in which the oxygen ion conductor 211 and the hydrogen ion conductor 212 are joined and the reference electrode 213 is formed on the oxygen ion conductor 211 and the sensing electrode 214 is formed on the hydrogen ion conductor 212 was used as the sensor unit 20. As the oxygen ion conductor 211 and the hydrogen ion conductor 212, Yttria stabilized zirconia (YSZ) and the $CaZr_{0.9}In_{0.1}O_{3-x}$ were respectively used, and the reference electrode 213 and the sensing electrode 214 were formed of platinum (Pt).

The sensor unit 20 was heated by using the heater unit 50 while measuring a temperature by connecting a thermocouple to the sensor unit 20, and changes in temperature and electromotive force with time were measured. As can be seen in FIG. 9, the electromotive force continuously increases as the temperature increases. Further, a stable electromotive force of about 1.1 V was measured at a temperature of about at least 300°C.

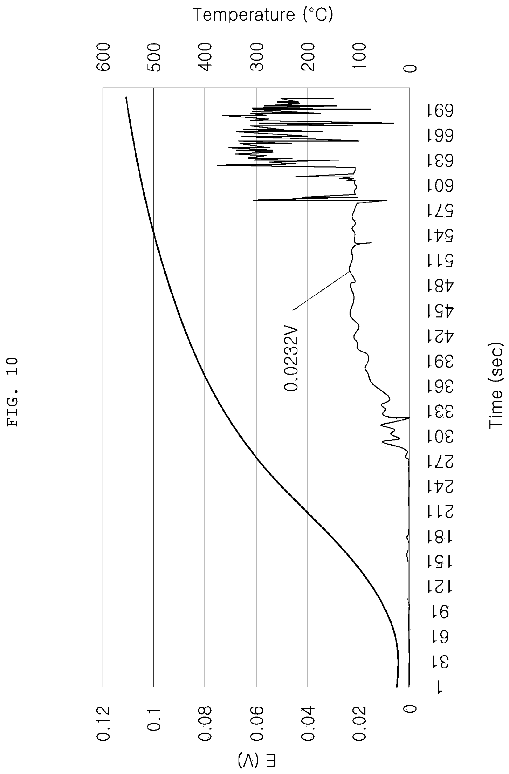

FIG. 10 is a graph illustrating a measuring result of the electromotive force E under the same condition by using a gas sensing device in which the connection passage 60 is not formed for comparison. Except that the gas sensing device used in FIG. 10 does not have the connection passage 60 and the first and second openings 61 and 62, the gas sensing device used in FIG. 10 was the same as the gas sensing device used in FIG. 9. Referring to FIG. 10, the electromotive force tends to increase as the temperature increases. However, comparing to the result in FIG. 9, it can be seen that the measured electromotive force value is very low and unstable.

From the result described above, it can be seen that the rapid and accurate measurement of the concentration of the gas may be realized when the gas sensing device of the present disclosure is used.

The present disclosure has been described with reference to the limited embodiments and drawings hereinabove, but it is apparent to those skilled in the art that various modifications can be made within the scope of the appended claims.

## Claims

1. A gas sensing device (1A, 1B) comprising:

    a housing (10) comprising an opening part (70) through which a target gas to be sensed enters an inner space thereof;
    the inner space (30) of the housing (10) is in communication with external air only through the opening part (70),
    a sensor unit (20) disposed in the inner space (30) of the housing (10);
    **characterised in that**
    a connection passage (60) connecting a first opening (61) and a second opening (62) that are formed in the housing such that the first opening (61) and the second opening (62) are open toward the inner space of the housing (10), and
    wherein the inner space (30) of the housing (10) comprises a first inner space (31) between the sensor unit (20) and the opening part (70) and a second inner space (32) that is a space except for the first inner space (31), the first opening (61) is open toward the first inner space (31), and the second opening (62) is open toward the second inner space (32).

2. The gas sensing device of claim 1, further comprising a heater unit (50) for heating the sensor unit (20) to a sensing temperature.

3. The gas sensing device of claim 1, wherein the housing (10) is formed in a hollow tubular shape such that the opening part (70) is formed in a first end portion of the housing (10) in a longitudinal direction, the sensor unit (20) is disposed in the inner space (30) of the housing (10) while the sensor unit (20) is in a state of being fixed to a first end portion of a frame (22) in a longitudinal direction, the frame (22) having a diameter smaller than an inner diameter of the housing (10), and a second end portion of the frame in the longitudinal direction is gas-tightly fixed to the housing (10).

4. The gas sensing device of claim 3, wherein a space between the frame (22) and an inner wall of the housing (10) forms the second inner space (32), and a circulation path circulating through the first inner space (30), the first opening (11), the connection passage (60), the second opening (62), and the second inner space (32) is formed.

5. The gas sensing device of claim 1, wherein the housing (10) is formed in a hollow tubular shape such that the opening part (70) is formed in a first end portion of the housing (10) in a longitudinal direction, a second end portion of the housing (10) in the longitudinal direction is blocked by a cover part (12), and the sensor unit (20) is disposed in the inner space (30) of the housing (10) while the sensor unit (20) is in a state of being coupled to an inner wall of the housing (10).

6. The gas sensing device of claim 5, wherein a gap exists between the sensor unit (20) and the inner wall of the housing (10), and a circulation path circulating through the first inner space (31), the first opening (61), the connection passage (60), the second opening (62), and the second inner space (32) is formed.

7. The gas sensing device of claim 1, wherein the sensor unit (20) comprises a hydrogen sensor element.

8. The gas sensing device of claim 7, wherein the hydrogen sensor element comprises:

    a solid electrolyte;

a sensing electrode (214) formed on a first surface of the solid electrolyte in a direction toward the opening part (70); and

a reference electrode (213) formed on a second surface of the solid electrolyte, wherein the second surface is an opposite surface of the electrolyte to the first surface,

wherein the first opening (61) is positioned between the sensing electrode (214) and the opening part (70).

9. The gas sensing device of claim 1, wherein

the inner space (30) is formed in a hollow tubular shape, the housing (10) being configured such that a lower end portion of the housing (10) in a longitudinal direction is open toward the inner space (30), thereby forming the opening part (70)

the sensor unit (20) is disposed at a position of the inner space (30), the position being spaced apart from both an upper end portion and the lower end portion of the housing (10) in the longitudinal direction by a predetermined distance, the sensor unit (20) comprising a sensing electrode (213) formed to face the opening part (70); and

a heater unit (50) is provided to heat the sensor unit (20) to a sensing temperature, and

the inner space (30) of the housing (10) comprises the first inner space (31) and the second inner space (32) that are respectively positioned below and above the sensing electrode (214) of the sensor unit (20) on the basis of the sensing electrode (214) of the sensor unit (20), and

the connection passage (60) connecting the first opening (61) and the second opening (62) forms a circulation path in which the target gas to be sensed enters through the opening part (70) and which is circulated through the first inner space (31), the first opening (61), the connection passage (60), the second opening (62), and the second inner space (32).

10. The gas sensing device of claim 9, wherein the sensor unit (20) is disposed at the inner space (30) while the sensor unit (20) is in a state of being fixed to a lower end portion of a frame (22) which is formed in a hollow tubular shape and which has a diameter smaller than an inner diameter of the housing (10), an inner portion of the frame (22) is exposed to external air while the inner portion of the frame (22) is isolated from the inner space (30) of the housing (10), and the sensor unit (20) further comprises a reference electrode (213) that is exposed to the external air through the inner portion of the frame (22).

11. The gas sensing device of claim 9, wherein the sensor unit (20) is disposed at the inner space (30) in a manner that a partial area of a border of the sensor unit (20) is coupled to an inner wall of the housing (10) through a coupling part (23), and a gap is formed at a portion which is a position where the coupling part (23) is not formed and which is positioned between the sensor unit (20) and the inner wall of the housing (10), so that the first inner space (31) and the second inner space (32) are in communication with each other through the gap.

**Patentansprüche**

1. Gaserfassungsvorrichtung (1A, 1B), umfassend:

ein Gehäuse (10) mit einem Öffnungsteil (70), durch den ein zu erfassendes Zielgas in einen Innenraum desselben eintritt;

wobei der Innenraum (30) des Gehäuses (10) nur durch den Öffnungsteil (70) mit Außenluft in Verbindung steht,

eine Sensoreinheit (20), die in dem Innenraum (30) des Gehäuses (10) angeordnet ist;

**dadurch gekennzeichnet, dass**

ein Verbindungsdurchgang (60) eine erste Öffnung (61) und eine zweite Öffnung (62) verbindet, die in dem Gehäuse so ausgebildet sind, dass die erste Öffnung (61) und die zweite Öffnung (62) zu dem Innenraum des Gehäuses (10) hin offen sind, und

wobei der Innenraum (30) des Gehäuses (10) einen ersten Innenraum (31) zwischen der Sensoreinheit (20) und dem Öffnungsteil (70) und einen zweiten Innenraum (32) umfasst, der ein Raum außer dem ersten Innenraum (31) ist, wobei die erste Öffnung (61) zu dem ersten Innenraum (31) hin offen ist und die zweite Öffnung (62) zu dem zweiten Innenraum (32) hin offen ist.

2. Gaserfassungsvorrichtung nach Anspruch 1 umfasst ferner eine Heizeinheit (50) zum Erwärmen der Sensoreinheit (20) auf eine Messtemperatur.

3. Gaserfassungsvorrichtung nach Anspruch 1, wobei das Gehäuse (10) in einer hohlen rohrförmigen Form ausgebildet ist, so dass der Öffnungsteil (70) in einem ersten Endabschnitt des Gehäuses (10) in einer Längsrichtung ausgebildet ist, die Sensoreinheit (20) in dem Innenraum (30) des Gehäuses (10) angeordnet ist, während sich die Sensoreinheit (20) in einem Zustand befindet, in dem sie an einem ersten Endabschnitt eines Rahmens (22) in einer Längsrichtung befestigt ist, wobei der Rahmen (22) einen Durchmesser aufweist, der kleiner als ein Innendurchmesser des Gehäuses (10) ist, und ein zweiter Endabschnitt des Rahmens in der Längsrichtung gasdicht an dem Gehäuse (10) befestigt ist.

4. Gaserfassungsvorrichtung nach Anspruch 3, wobei ein Raum zwischen dem Rahmen (22) und einer Innenwand des Gehäuses (10) den zweiten Innenraum (32) bildet und ein Zirkulationsweg, der durch den ersten Innenraum (30), die erste Öffnung (11), den Verbindungsdurchgang (60), die zweite Öffnung (62) und den zweiten Innenraum (32) zirkuliert, gebildet wird.

5. Gaserfassungsvorrichtung nach Anspruch 1, wobei das Gehäuse (10) in einer hohlen röhrenförmigen Form ausgebildet ist, so dass der Öffnungsteil (70) in einem ersten Endabschnitt des Gehäuses (10) in einer Längsrichtung ausgebildet ist, ein zweiter Endabschnitt des Gehäuses (10) in der Längsrichtung durch einen Deckelteil (12) blockiert ist und die Sensoreinheit (20) in dem Innenraum (30) des Gehäuses (10) angeordnet ist, während die Sensoreinheit (20) in einem Zustand ist, in dem sie mit einer Innenwand des Gehäuses (10) gekoppelt ist.

6. Gaserfassungsvorrichtung nach Anspruch 5, wobei ein Spalt zwischen der Sensoreinheit (20) und der Innenwand des Gehäuses (10) besteht und ein Zirkulationspfad, der durch den ersten Innenraum (31), die erste Öffnung (61), den Verbindungsdurchgang (60), die zweite Öffnung (62) und den zweiten Innenraum (32) zirkuliert, gebildet wird.

7. Gaserfassungsvorrichtung nach Anspruch 1, wobei die Sensoreinheit (20) ein Wasserstoffsensorelement umfasst.

8. Gaserfassungsvorrichtung nach Anspruch 7, wobei das Wasserstoffsensorelement Folgendes umfasst:

einen festen Elektrolyten;
eine Sensorelektrode (214), die auf einer ersten Oberfläche des Festelektrolyten in Richtung des Öffnungsteils (70) ausgebildet ist; und
eine Referenzelektrode (213), die auf einer zweiten Oberfläche des Festelektrolyten ausgebildet ist,
wobei die zweite Oberfläche eine der ersten Oberfläche gegenüberliegende Oberfläche des Elektrolyten ist,
wobei die erste Öffnung (61) zwischen der Sensorelektrode (214) und dem Öffnungsteil (70) angeordnet ist.

9. Gaserfassungsvorrichtung nach Anspruch 1, wobei

der Innenraum (30) in einer hohlen röhrenförmigen Form ausgebildet ist, wobei das Gehäuse (10) so konfiguriert ist, dass ein unterer Endabschnitt des Gehäuses (10) in einer Längsrichtung zu dem Innenraum (30) hin offen ist, wodurch der Öffnungsteil (70) gebildet wird;
die Sensoreinheit (20) an einer Position des Innenraums (30) angeordnet ist, wobei die Position sowohl von einem oberen Endabschnitt als auch von dem unteren Endabschnitt des Gehäuses (10) in der Längsrichtung um einen vorbestimmten Abstand beabstandet ist, wobei die Sensoreinheit (20) eine Sensorelektrode (213) umfasst, die so ausgebildet ist, dass sie dem Öffnungsteil (70) gegenüberliegt; und
eine Heizeinheit (50) vorgesehen ist, um die Sensoreinheit (20) auf eine Fühltemperatur zu erwärmen, und der Innenraum (30) des Gehäuses (10) den ersten Innenraum (31) und den zweiten Innenraum (32) umfasst, die jeweils unterhalb und oberhalb der Sensorelektrode (214) der Sensoreinheit (20) auf der Basis der Sensorelektrode (214) der Sensoreinheit (20) angeordnet sind,
und
der Verbindungsdurchgang (60), der die erste Öffnung (61) und die zweite Öffnung (62) verbindet, einen Zirkulationspfad bildet, in den das zu erfassende Zielgas durch den Öffnungsteil (70) eintritt und der durch den ersten Innenraum (31), die erste Öffnung (61), den Verbindungsdurchgang (60), die zweite Öffnung (62) und den zweiten Innenraum (32) zirkuliert wird.

10. Gaserfassungsvorrichtung nach Anspruch 9, wobei die Sensoreinheit (20) in dem Innenraum (30) angeordnet ist, während sich die Sensoreinheit (20) in einem Zustand befindet, in dem sie an einem unteren Endabschnitt eines Rahmens (22) befestigt ist, der in einer hohlen, rohrförmigen Form ausgebildet ist und der einen Durchmesser aufweist, der kleiner als ein Innendurchmesser des Gehäuses (10) ist, ein innerer Abschnitt des Rahmens (22) der Außenluft ausgesetzt ist, während der innere Abschnitt des Rahmens (22) von dem Innenraum (30) des Gehäuses (10) isoliert ist, und die Sensoreinheit (20) ferner eine Referenzelektrode (213) umfasst, die der Außenluft durch den inneren Abschnitt des Rahmens (22) ausgesetzt ist.

11. Gaserfassungsvorrichtung nach Anspruch 9, wobei die Sensoreinheit (20) in dem Innenraum (30) so angeordnet ist, dass ein Teilbereich eines Randes der Sensoreinheit (20) mit einer Innenwand des Gehäuses (10) über ein Kupplungsteil (23) gekoppelt ist, und ein Spalt an einem Abschnitt ausgebildet ist, der eine Position ist, an der das Kopplungsteil (23) nicht ausgebildet ist und der zwischen der Sensoreinheit (20) und der Innenwand des Gehäuses (10) angeordnet ist, so dass der erste Innenraum (31) und der zweite Innenraum (32) durch den Spalt miteinander in Verbindung stehen.

**Revendications**

1. Dispositif de détection de gaz (1A, 1B) comprenant:

   un boîtier (10) comprenant une partie d'ouverture (70) à travers laquelle un gaz cible à détecter pénètre dans son espace intérieur ;
   l'espace intérieur (30) du boîtier (10) est en communication avec l'air extérieur uniquement à travers la partie d'ouverture (70),
   une unité de détection (20) disposée dans l'espace intérieur (30) du boîtier (10) ;

   **caractérisé en ce que**

   un passage de connexion (60) reliant une première ouverture (61) et une seconde ouverture (62) qui sont formées dans le boîtier de sorte que la première ouverture (61) et la seconde ouverture (62) sont ouvertes vers l'espace intérieur du boîtier (10), et
   dans lequel l'espace intérieur (30) du boîtier (10) comprend un premier espace intérieur (31) entre l'unité de capteur (20) et la partie d'ouverture (70) et un deuxième espace intérieur (32) qui est un espace à l'exception du premier espace intérieur (31), la première ouverture (61) est ouverte vers le premier espace intérieur (31), et la deuxième ouverture (62) est ouverte vers le deuxième espace intérieur (32).

2. Le dispositif de détection de gaz de la revendication 1, comprenant en outre une unité de chauffage (50) pour chauffer l'unité de détection (20) à une température de détection.

3. Le dispositif de détection de gaz de la revendication 1, dans lequel le boîtier (10) est formé dans une forme tubulaire creuse de sorte que la partie d'ouverture (70) est formée dans une première partie d'extrémité du boîtier (10) dans une direction longitudinale , l'unité de détection (20) est disposée dans l'espace intérieur (30) du boîtier (10) alors que l'unité de détection (20) est en état d'être fixée à une première partie d'extrémité d'un cadre (22) dans une direction longitudinale, le cadre (22) ayant un diamètre inférieur à un diamètre intérieur du boîtier (10), et une deuxième partie d'extrémité du cadre dans la direction longitudinale est fixée de manière étanche au gaz au boîtier (10).

4. Le dispositif de détection de gaz de la revendication 3, dans lequel un espace entre le cadre (22) et une paroi intérieure du boîtier (10) forme le second espace intérieur (32), et un chemin de circulation traversant le premier espace intérieur (30), la première ouverture (11), le passage de connexion (60), la seconde ouverture (62), et le second espace intérieur (32) est formé.

5. Le dispositif de détection de gaz de la revendication 1, dans lequel le boîtier (10) est formé dans une forme tubulaire creuse de telle sorte que la partie d'ouverture (70) est formée dans une première partie d'extrémité du boîtier (10) dans une direction longitudinale, une deuxième partie d'extrémité du boîtier (10) dans la direction longitudinale est bloquée par une partie de couvercle (12), et l'unité de capteur (20) est disposée dans l'espace intérieur (30) du boîtier (10) alors que l'unité de capteur (20) est en état d'être couplée à une paroi intérieure du boîtier (10).

6. Le dispositif de détection de gaz de la revendication 5, dans lequel un espace existe entre l'unité de capteur (20) et la paroi intérieure du boîtier (10), et un chemin de circulation traversant le premier espace intérieur (31), la première ouverture (61), le passage de connexion (60), la seconde ouverture (62), et le second espace intérieur (32) est formé.

7. Le dispositif de détection de gaz de la revendication 1, dans lequel l'unité de détection (20) comprend un élément de détection d'hydrogène.

8. Le dispositif de détection de gaz de la revendication 7, dans lequel l'élément de détection d'hydrogène comprend :

un électrolyte solide ;
une électrode de détection (214) formée sur une première surface de l'électrolyte solide en direction de la partie d'ouverture (70) ; et
une électrode de référence (213) formée sur une deuxième surface de l'électrolyte solide,
dans laquelle la seconde surface est une surface de l'électrolyte opposée à la première surface,
la première ouverture (61) est placée entre l'électrode de détection (214) et la partie d'ouverture (70).

9. Le dispositif de détection de gaz de la revendication 1, dans lequel

l'espace intérieur (30) est formé dans une forme tubulaire creuse, le boîtier (10) étant configuré de telle sorte qu'une partie d'extrémité inférieure du boîtier (10) dans une direction longitudinale est ouverte vers l'espace intérieur (30), formant ainsi la partie d'ouverture (70) ;
l'unité de détection (20) est disposée à une position de l'espace intérieur (30), la position étant espacée à la fois d'une partie d'extrémité supérieure et d'une partie d'extrémité inférieure du boîtier (10) dans la direction longitudinale par une distance prédéterminée, l'unité de détection (20) comprenant une électrode de détection (213) formée pour faire face à la partie d'ouverture (70); et
une unité de chauffage (50) est prévue pour chauffer l'unité de détection (20) à une température de détection, et l'espace intérieur (30) du boîtier (10) comprend le premier espace intérieur (31) et le second espace intérieur (32) qui sont respectivement positionnés en dessous et au-dessus de l'électrode de détection (214) de l'unité de détection (20) sur la base de l'électrode de détection (214) de l'unité de détection (20), et
le passage de connexion (60) reliant la première ouverture (61) et la seconde ouverture (62) forme un chemin de circulation dans lequel le gaz cible à détecter entre par la partie d'ouverture (70) et qui circule à travers le premier espace intérieur (31), la première ouverture (61), le passage de connexion (60), la seconde ouverture (62) et le second espace intérieur (32).

10. Le dispositif de détection de gaz de la revendication 9, dans lequel l'unité de détection (20) est disposée dans l'espace intérieur (30) alors que l'unité de détection (20) est en état d'être fixée à une partie d'extrémité inférieure d'un cadre (22) qui est formé dans une forme tubulaire creuse et qui a un diamètre plus petit qu'un diamètre intérieur du boîtier (10), une partie intérieure du cadre (22) est exposée à l'air extérieur tandis que la partie intérieure du cadre (22) est isolée de l'espace intérieur (30) du boîtier (10), et l'unité de détection (20) comprend en outre une électrode de référence (213) qui est exposée à l'air extérieur à travers la partie intérieure du cadre (22).

11. Le dispositif de détection de gaz de la revendication 9, dans lequel l'unité de détection (20) est disposée dans l'espace intérieur (30) de manière à ce qu'une zone partielle d'un bord de l'unité de détection (20) soit couplée à une paroi intérieure du boîtier (10) par l'intermédiaire d'une pièce de couplage (23), et un espace est formé à une partie qui est une position où la pièce de couplage (23) n'est pas formée et qui est positionnée entre l'unité de capteur (20) et la paroi intérieure du boîtier (10), de sorte que le premier espace intérieur (31) et le second espace intérieur (32) sont en communication l'un avec l'autre par l'intermédiaire de l'espace.

FIG. 1

1A

FIG. 2

<u>1B</u>

FIG. 3

FIG. 4

FIG. 5

FIG. 6

FIG. 7

FIG. 8

FIG. 9

EP 4 113 096 B1

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101512189
- KR 20160011722
- US 2004018705 A1
- US 20180217020 A1